# EUROPEAN PATENT APPLICATION

(11) **EP 2 377 525 A1**
(43) Date of publication of application: **19.10.2011**
(21) Application number: 10382069.2
(22) Date of filing: 26.03.2010
(51) Int. Cl.: A61K 9/50, A61K 31/381

(54) **Duloxetine enteric pellets**

(71) Applicant: Laboratorios del Dr. Esteve S.A., 08041 Barcelona (ES)
(72) Inventor: Casadevall Pujals, Gemma, E-08019 Barcelona (ES); García Rovira, Monserrat, E-08301 Mataró (ES)
(74) Representative: ABG Patentes, S.L.

(57) **Abstract**

The present invention relates to pellets comprising a core with duloxetine, an intermediate non porous layer comprising one or more film forming water-soluble polymers, and an enteric layer comprising an enteric polymer different than polyvinyl acetate phthalate, to a process for preparing the same, to the use of the pellets for manufacturing a pharmaceutical dosage form, and to a dosage form containing them.

## Description

### FIELD OF THE INVENTION

The present invention relates to enteric pellets of duloxetine or pharmaceutically acceptable salts thereof and a method for their manufacture.

### BACKGROUND OF THE INVENTION

Duloxetine is a selective serotonin and norepinephrine reuptake inhibitor having the chemical name (+)-(S)-N-methyl-γ-(1-naphthyloxy)-2-thiophenepropylamine Duloxetine, sold as its hydrochloride salt, is disclosed in the European patent application EP 273 658.

Delayed release formulations are advantageous, as they prevent exposure of an acid sensitive active pharmaceutical ingredient (API) to the acidic environment of a patient's stomach, preventing degradation of the API and /or irritation of the patient's stomach.

Since duloxetine is an acid labile API, it is advisable to formulate it as an enteric formulation.

European patent application EP 0 693 282 A2 discloses a delayed release duloxetine formulation in the form of an enteric duloxetine coated pellet. The selected enteric coating polymer is hydroxypropylmethylcellulose acetate succinate (HPMCAS). When the enteric polymer is applied as an aqueous suspension, a problem in obtaining a uniform, coherent film often arises. It is important to assure that the suspension remains homogeneous, and that conditions responsible for the agglomeration of the polymer do not occur. In particular, it is advisable to cool the suspension of HPMCAS below 20°C before application, to cool the tubing and nozzle by pumping a little cold water through them before beginning to pump the suspension, and to use supply tubing with as small diameter as the spray rate will allow so that the suspension can be kept moving rapidly in the tubing. It is preferred to apply the enteric polymer as an aqueous solution whenever it is possible to do so. The dissolution of HPMCAS can be obtained by neutralizing the polymer, preferably with ammonia.

International patent application WO 2007/139886 discloses a duloxetine hydrochloride delayed release formulation comprising an inert core, a drug layer comprising duloxetine hydrochloride, a separating layer, an enteric layer comprising at least one of a methacrylic acid copolymer and hydroxypropyl methyl cellulose phthalate, and optionally a finishing layer. The enteric layer is applied to accomplish delayed release of the duloxetine hydrochloride primarily in the small intestine. Preferably, the enteric layer is substantially insoluble in acidic environments, such as the stomach, but is soluble in near-neutral environments, such as the small intestine. Thus, the formulation remains intact as it passes through the acid environment of the stomach, but dissolves and releases the duloxetine hydrochloride once it passes into the near-neutral environment of the small intestine. The enteric layer preferably contains a polymer that dissolves at pH of above about 5.5.

International patent application WO 2008/020286 relates to a delayed pharmaceutical composition comprising a core containing duloxetine and an enteric coat that comprises methacrylic acid copolymer (Eudragit^{®}). Methacrylic acid copolymer based enteric layers do not dissolve at pH lower than 6.

In order to begin the dissolution of the enteric layers at a lower pH, Eudragit^{®} can be chemically treated, for instance, by neutralising with NaOH, or by reaction of the free carboxylic groups of methacrylic acid copolymer to form esters, e.g. phthalates, acetates, etc. Even applying those treatments, the methacrylic acid copolymer does not begin to dissolve until pH higher than 5.

International patent application WO 2008/077939 relates to a pharmaceutical pellet composition comprising duloxetine or a pharmaceutically acceptable salt thereof, particularly, the hydrochloride salt, a separating layer comprising a water soluble inorganic salt in the form of crystals, and an enteric layer. Pellets described in examples 2 and 4 contain an intermediate layer comprising hypromellose and an enteric layer comprising polyvinyl acetate phthalate.

Patent application US 2008/0226711 refers to solid oral pharmaceutical compositions comprising a core comprising duloxetine, optional separating coat and an enteric coat. Pellets comprising cores covered with duloxetine hydrochloride, a separating layer comprising HPMC and an enteric layer comprising polyvinyl acetate phthalate and diethyl phthalate are described.

Patent US 5 776 969 discloses pellets containing sucrose-starch non-pareils with a coat comprising duloxetine, an intermediate layer comprising HPMC, an enteric layer comprising HPMC acetate succinate and a finishing layer. Polyvinyl acetate phthalate is mentioned among others suitable enteric polymers.

### FIGURES

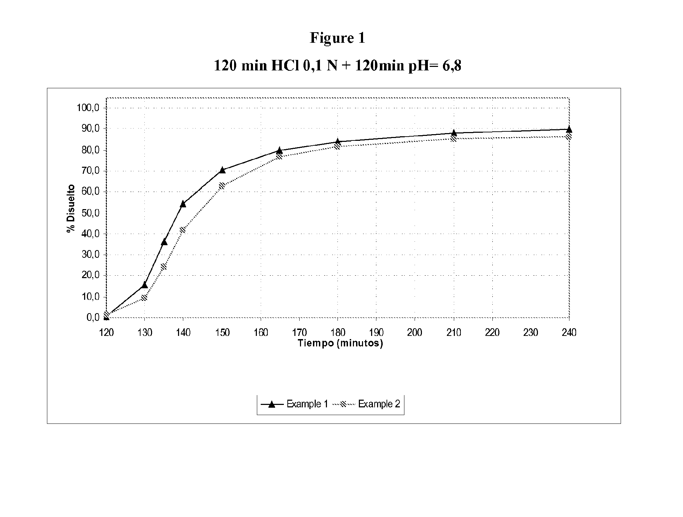

Figure 1: graphic showing profiles of dissolution of formulations corresponding to examples 1.

### BRIEF DESCRIPTION OF TE INVENTION

There is still a need in the art to prepare enteric formulations of duloxetine with improved stability with respect to degradation and which provide a better and quicker absorption of said active principle. Moreover, it should be desirable that this kind of formulations is manufactured using simple and safe processes.

The inventors have developed an enteric pharmaceutical formulation of duloxetine which allows dissolution of the active principle duloxetine at a pH of approximately 6, maintaining their protection against acidity. This is very relevant since solubility of duloxetine is higher at around this pH than at any other physiologically acceptable pH value. In consequence, when this highly soluble duloxetine reaches the small intestine at said pH, it will be better and quicker absorbed.

The enteric pharmaceutical formulations of duloxetine according to the present invention, which take the form of pellets, achieve unexpected advantage over the formulations of the prior art by including in the formulation of the pellet an enteric coating layer comprising an enteric polymer different than polyvinyl acetate phthalate and a nonporous intermediate layer which acts as a barrier in order to stop proton transfer from the external medium to the core.

Thus, according to one aspect, the present invention is directed to a pellet comprising:
a) a core comprising duloxetine or a pharmaceutically acceptable salt thereof and pharmaceutically acceptable excipients,
b) an intermediate non porous layer comprising one or more film forming water-soluble polymers,
c) an enteric coating layer comprising an enteric polymer different than polyvinyl acetate phthalate, and
d) an optional finishing layer.

A second aspect of the invention is the use of a pellet as described above for manufacturing a pharmaceutical dosage form.

A third aspect of the invention is a pharmaceutical dosage form which comprises a pellet as described above.

A fourth aspect of the invention is a process for manufacturing a pellet as described above comprising:
a) preparing a core comprising duloxetine or a pharmaceutically acceptable salt thereof,
b) coating said core with an intermediate non porous layer comprising one or more film forming water-soluble polymers, and
c) coating said intermediate layer with an enteric coating layer comprising an enteric polymer different than polyvinyl acetate phthalate.

The pellet of the invention shows a satisfactory dissolution profile which is obtained using an enteric polymer and polymers useful to prepare the non porous intermediate layer which can be found in the commerce and used without further treatment.

The process for manufacturing said pellets can be carried out in conventional industrial equipments. Finally, since only aqueous mediums are used during the process, the problems due to residual solvent restriction in the final product and environment safety are thus avoided.

### DETAILED DESCRIPTION OF THE INVENTION

The pellet of the invention contains duloxetine or a pharmaceutically acceptable salt thereof as active principle. The pharmaceutically acceptable salt most commonly used is the hydrochloride salt. Therefore, in a particular embodiment, the pellets comprise duloxetine hydrochloride.

The core of the pellet described above may comprise a monolithic particle comprising duloxetine or its pharmaceutical salts or may be formed by an inert bead which is covered with a layer comprising the active principle. Preferentially, the core may be made from an inert bead which is covered by a layer comprising the active principle and pharmaceutically acceptable excipients.

The inert bead is inert with regard both to duloxetine and to the other excipients in the pellet, and with regard to the patient who will ingest the pellet. Such inert bead is conventionally used in pharmaceutical techniques.

The bead may be prepared from materials such as, e. g. starch, sucrose, microcrystalline cellulose, and the like. The size of the beads depends on the desired size of the pellet to be manufactured or further processed.

The layer containing the active principle may include excipients commonly used in pharmaceutical formulations that do not interact adversely with duloxetine and its salts.

In a particular embodiment, the core of the pellet comprises an inert bead and said inert bead is covered by a layer comprising duloxetine, or a pharmaceutically acceptable salt, and pharmaceutically acceptable excipients.

As used herein, the term "pharmaceutically acceptable" means approved by a regulatory agency of the Federal or a state government or listed in the U.S. Pharmacopeia or other generally recognized pharmacopeia for use in animals, and more particularly in humans.

Preferably, the pharmaceutically acceptable excipients are selected from diluents, binders, glidants, coating agents, and anti-static agents. Inert substances such as talc, kaolin, and titanium dioxide, lubricants such as magnesium stearate, finely divided silicon dioxide, crospovidone, and β-lactose, binders as hydroxypropyl methyl cellulose may be used.

The pellet comprises an intermediate non porous layer between the core and the enteric coating. This intermediate layer provides stability by inhibiting direct contact of components of the core and the enteric polymer in the enteric coating. It also provides protection to the core during its passage from the stomach to the intestines. Said intermediate layer is compatible with duloxetine and the enteric coating.

The most important role of the intermediate non porous layer is to act as a barrier that avoids the proton's transfer from the external medium to the core. This migration would affect negatively the stability of duloxetine. In order to achieve this, the intermediate protective layer should also be readily soluble in water as it has to be dissolved once the enteric coating is dissolved in the neutral or nearly neutral pH conditions of the intestine, and thus, duloxetine has to be released fast. At the same time the intermediate layer should be non porous to stop proton transfer from the external medium to the core.

The term "water soluble polymers" refers to polymers well known in the Pharmaceutical Industry, for example, from Remington, "The Science and Practice of Pharmacy", 21st Edition, Chapter 21, page 305. "

The term "non porous" with reference to the intermediate layer of the invention should be understood as that it prevents the migration of the protons from the medium to the core of the pellets. Said characteristic is demonstrated through the dissolution profile of duloxetine obtained using the method described in the 2009 U.S. Pharmacopeia 32st editions. Under those conditions the dissolution of duloxetine is more than 85 % within of a period of 210 min from the beginning of the test indicating duloxetine is not degraded by migration of the protons.

This layer comprises one or more film forming water-soluble polymer e.g., polyvinyl alcohol (PVA), copovidone (copolyvidone), methyl cellulose, and polyvinylpyrrolidone (PVP), etc.

In a particular embodiment of the invention the intermediate non porous layer in the pellet comprises copovidone. Copovidone is a copolymer of 1-vinyl-2-pyrrolidone and vinyl acetate

In other particular embodiment the intermediate non porous layer in the pellet comprises a polyvinyl alcohol polymer.

In other particular embodiment the water-soluble polymer of the intermediate layer is present in an amount ranging from 4 to 9% by weight of the pellet.

This intermediate layer may contain other pharmaceutically acceptable excipients such as binder agents: e.g., sucrose; thickening agents: e.g., talc, glycerol monoestearate; surfactants: e.g. polysorbates; plasticizers: e.g. triethyl citrate, polyethylene glycol.

In other particular embodiment the intermediate non porous layer in the pellet further comprises other pharmaceutically acceptable excipients.

Examples of said film forming water soluble polymers that may be obtained from the commerce are Kollidon^{®} VA 64 (registered trademark of BASF Corporation) and Opadry^{®} II 85FI9250 clear (registered trademark of Colorcon, Inc)

Kollidon^{®} VA 64 is a vinylpyrrolidone-vinyl acetate copolymer that is soluble both in water and in alcohol. It is used as a dry binder in tableting, as a granulating auxiliary and as a film-forming agent in the pharmaceutical industry. Ratio vinylpyrrolidone:vinyl acetate in the copolymer is 6:4.

Opadry^{®} II 85FI9250 clear contains the following ingredients: polyvinyl alcohol, talc, polyethylene glycol and polysorbate 80. The quantitative composition is the following:

| | |
|---|---|
| Polyvinyl alcohol | 52,26 % w/w |
| Talc | 30,00 % w/w |
| Polyethylene glycol | 17.74 % w/w |
| Polysorbate 80 | 3,00 % w/w |

Other pharmaceutically acceptable excipients may be sucrose, talc, titanium dioxide, etc.

The enteric coating comprises an enteric polymer different than polyvinyl acetate phthalate.

As used herein, the term "enteric polymer" means a pH dependent polymer that is substantially insoluble at a low pH (lower than 4), increases its dissolution to a pH of approximately 5, and is soluble in near-neutral environments, such as the small intestine.

As used in the present patent application, the term "an enteric coating layer comprising an enteric polymer different than polyvinyl acetate phthalate" means that the enteric polymer in the enteric coating layer comprises suitable pH-dependent polymer.

In a another embodiment of the invention the suitable pH-dependent polymer is selected from cellulose acetate phthalate, cellulose acetate succinate, methylcellulose phthalate, hydroxypropyl methylcellulose phthalate, ethylhydroxycellulose phthalate, polymethacrylates such as methacrylic acid copolymer, etc.

In a particular embodiment of the invention the enteric coating layer comprises methacrylic acid copolymer. This copolymer increases its solubility already at a pH value of 6.

This coating layer may contain other pharmaceutically acceptable excipients such as thickening agents: e.g., talc, glycerol monoestearate; surfactants: e.g. polysorbates; plasticizers: e.g. triethyl citrate. Therefore, in a particular embodiment, the enteric coating layer further comprises pharmaceutically acceptable excipients.

In other particular embodiment the excipient is a plasticizer, more particularly triethylcitrate; and even more particularly the triethylcitrate is present in amount ranging from 0,4 to 3% by weight of the pellet.

Other particular embodiment concerns pharmaceutical dosage forms wherein the enteric polymer is present in amount ranging from 5 to 15% by weight of the pellet.

An optional finishing layer may be applied on the top of the enteric layer in order to aid in the handling of the formulation. Thus, said finishing layer reduces the static charge and prevents the pellets to stick together and easy further processing unit operations. The layer preferably comprises a coating agent and, optionally one or more pharmaceutically acceptable excipients. Preferably, the coating agent is hydroxypropylmethylcellulose, polyvinyl pyrrolidone, and like. The additional pharmaceutically acceptable excipients can be e.g. thickening agents, glidants, stabilizers, and colouring maters. More preferably, the additional pharmaceutically acceptable excipients are selected from talc, colloidal silicon dioxide, and titanium dioxide.

Solid pharmaceutical dosage forms comprising or made of pellets are well-known, for example tablets or capsules.

Tablets, particularly orally disintegrable tablets, are advantageously used in cases where administration without water is necessary, in cases of administration to patients who have difficulty in swallowing tablets or in cases of administration to the aged or to children where there is a fear of blocking the throat. The pharmaceutically active ingredient is comprised in the tablet in the form of fine granules, more preferably the fine granules are pellets.

Pellets also may be filled into hard or soft capsules (e.g. gelatine capsules) or provided in a sachet.

Therefore, another aspect of the invention is the use of the pellet as described before for manufacturing a pharmaceutical dosage form.

Another aspect of the invention is a pharmaceutical dosage form comprising a pellet as described before. Particularly, the pharmaceutical dosage form is a tablet or a capsule.

Processes for manufacturing and coating a pellet as described above are known in the pharmaceutical industry.

If the core of the pellets is a monolithic particle comprising the active principle, it may be prepared for example, by the granulation techniques. The core may be prepared by mixing the duloxetine into a mass of pharmaceutical excipients, moistening the mass with water or an organic solvent in a high shear granulator to form a homogeneous wet mass. Next, the wet mass is extruded to form extrudates which can be spheronized. Hot melt extrusion techniques are also an alternative.

If the core comprises an inert bead, the bead may be coated with duloxetine or a pharmaceutical salt thereof, using methods known in the technique.

The layer comprising the enteric polymer, e.g., the enteric coating, may be formed by applying an aqueous suspension of the enteric polymer and optional pharmaceutically acceptable excipients.

Pellets having an intermediate layer may be manufactured by spraying solutions or suspensions of a polymeric material and dusting in the filler. Preferentially, the filler is thoroughly dispersed as a suspension in the solution of polymeric, material, and the suspension is sprayed on the core and dried.

Another aspect of the invention is a process for manufacturing a pellet as described before, which comprises the following steps:
a) preparing a core comprising duloxetine or a pharmaceutically acceptable salt thereof,
b) coating said core with an intermediate non porous layer comprising one or more film forming water-soluble polymers, and
c) coating said intermediate layer with an enteric coating layer comprising an enteric polymer different than polyvinyl acetate phthalate.

In a particular embodiment the intermediate non porous layer comprises copovidone or polyvinyl alcohol polymer.

In a particular embodiment the enteric coating layer comprises methacrylic acid copolymer.

In other particular embodiment the process further comprises to apply a finishing layer after performing step c).

When the core is obtained from an inert bead the layer containing the active compound may be formed using a powder coating process. The beads are moistened with a sticky liquid or binder, duloxetine or a pharmaceutical salt thereof, and optionally, excipients such as, plasticizers, binders, anti-taking agents, stabilizers or opacifying agents are added as a powder, and the mixture is sprayed onto the beads and then the beads are dried. The process can be carried out in conventional coating pans, in a fluidised bed equipment, or in a rotating plate equipment.

Preferably, the layer of active principle is built on the bead by spraying an aqueous solution of the duloxetine or a pharmaceutical salt thereof in which the pharmaceutical excipients are dissolved or suspended. Duloxetine or a pharmaceutical salt thereof is dissolved in hot water to a temperature between 30°C and 50°C, preferentially, between 35°C and 40°C. The pharmaceutical excipients are added.

In the present application, the range expressions such as "between 30°C and 50°C", and similar expressions, should be understood as including the extremes of the range.

In another particular embodiment of the process described above the core prepared in step a) comprises:
i) dissolving duloxetine or a pharmaceutically acceptable salt thereof in water, and
ii) spraying the solution obtained in i) on an inert bead,
wherein steps i) and ii) are carried out at a temperature between 30°C and 50°C. Preferably, said temperature is between 35°C and 40°C.

In a more particular embodiment step ii) is carried out in a fluid bed apparatus.

### EXAMPLES

The pellet of the invention and those of comparative examples were prepared following the scheme describe bellow.

### Part I- Core

Inert Sugar spheres were placed in a fluid bed dryer. The average diameter of the sugar spheres was 710-850 µm.

### Part II- Drug layer: Film Coating 1 (FC1)

Duloxetine hydrochloride, hydroxypropyl methylcellulose and talc were mixed in hot water (35°C-50°C). Duloxetine was fully dissolved.

The resulting solution was sprayed, while mixing and maintaining its temperature between 35°C and 50°C, onto the core in the fluid bed dryer with a 1,2 mm nozzle. The inlet air temperature was 52°C, the flap was 175 m³/hr, and the spray rate was 5 to 12 g/min. The pellets covered by drug layer FC1 (FC1 pellets) were then dried in the fluid bed dryer for an additional time at 40°C to form drug-coated pellets.

Optionally, a pre-film coating layer was prepared using hydroxypropyl methylcellulose and water and sprayed onto the sugar spheres before applying the drug layer film coating (FC1).

### Part III- Intermediate layer: Film coating 2 (FC2)

Kollidon^{®} VA 64, sucrose (optionally) and talc were mixed in purified water in a mixer until a homogeneous dispersion was obtained. The resulting suspension was sprayed onto the drug-coated FC1 Pellets in the fluid bed dryer through a 1,2 mm nozzle. The inlet air temperature was 52°C, the flap was 175 m³/hr, and the spray rate was 5 to 12 g/min. The FC2 pellets were dried in the fluid bed dryer for an additional time at 40°C to form sub-coated pellets.

Alternatively, a seal layer Film Coating 2 or FC2 has been prepared using Opadry^{®} II 85F18250 clear instead of Kollidon^{®}VA 64 and talc.

### Part IV-Enteric layer: Film Coating 3 (FC3)

Plasticizer (triethyl citrate), titanium dioxide and thickening agent (talc, glycerol monoestearate) of this film coating were mixed in purified water until an homogeneous dispersion was obtained. The resulting solution was mixed with enteric film former in a homogenizer for 30 minutes. The resulting suspension was sprayed onto the sub-coated FC2 pellets in the fluid bed dryer through a 1,2 mm nozzle. The inlet air temperature was 39°C, the flap was 150 m³/hr, and the spray rate was 5 to 8 g/min. The FC3 pellets were then dried in the fluid bed dryer for an additional time at 40°C to form enteric-coated pellets.

### Example 1

Duloxetine hydrochloride delayed release pellets containing an enteric layer of methacrylic acid copolymer and Kollidon VA64 in the intermediate layer. Each film coating (FC) suspension was made and sprayed over the preceding layer in a fluid bed apparatus according with the following components:

| **Coat** | **Ingredient** | **Concentration (%w/w)** |
|---|---|---|
| Core | Sugar spheres | 47,03 |
| FC 1 | Duloxetine HCl | 20,00 |
| | Hydroxypropyl methylcellulose | 8,00 |
| | Talc | 6,55 |
| | Purified water* | 100,00 |
| FC 2 | Kollidon VA 64 (copovidone) | 7,50 |
| | Talc | 1,00 |
| | Purified water* | 48,00 |
| FC 3 | Eudragit L30-D55 (methacrylic acid copolymer) | 7,60 |
| | Titanium dioxide | 0,80 |
| | Triethylcitrate | 0,76 |
| | Talc | 0,76 |
| | Purified water* | 385,00 |

| | | |
|---|---|---|
| *Water is evaporated during the process | | |

### Example 2

Duloxetine hydrochloride delayed release pellets containing a pre-film coating layer of hydroxypropyl methylcellulose and water, an enteric layer of methacrylic acid copolymer and Kollidon VA64 and sucrose in the intermediate layer. Each film coating (FC) suspension was made and sprayed over the preceding layer in a fluid bed apparatus according with the following components:

| **Coat** | **Ingredient** | **Concentration (%w/w)** |
|---|---|---|
| Core | Sugar spheres | 41,90 |
| Pre-FC 1 | Hydroxypropyl methylcellulose | 2,10 |
| | Talc | 0,30 |
| | Purified water | 14,0 |
| FC 1 | Duloxetine HCl | 20,00 |
| | Hydroxypropyl methylcellulose | 8,00 |
| | Talc | 6,55 |
| | Purified water* | 100,00 |
| FC 2 | Kollidon VA 64 (copovidone) | 8,50 |
| | Sucrose | 2,00 |
| | Talc | 1,50 |
| | Purified water* | 67,50 |
| FC 3 | Eudragit L30-D55 (methacrylic acid copolymer) | 7,00 |
| | Titanium dioxide | 0,75 |
| | Triethylcitrate | 0,70 |
| | Talc | 0,70 |
| | Purified water* | 385,00 |

| | | |
|---|---|---|
| *Water is evaporated during the process | | |

### Results of dissolution tests

Figure 1 shows the results of dissolution tests performed with pellets according to example 1 and 2. The release of the duloxetine from the pharmaceutical formulation was tested in USP apparatus 1 (rotating basket) at 37°C and 100 rpm with 2 hours in 0.1 N HCl (gastric challenge) followed by 120 minutes in phosphate buffer (50mM, pH 6.8). Both examples show a dissolution profile of duloxetine about 85% or more of said compound within of a period of 210 minutes from the beginning of the assay.

## Claims

1. Pellet comprising:
a) a core comprising duloxetine or a pharmaceutically acceptable salt thereof and pharmaceutically acceptable excipients,
b) an intermediate non porous layer comprising one or more film forming water-soluble polymers,
c) an enteric coating layer comprising an enteric polymer different than polyvinyl acetate phthalate, and
d) an optional finishing layer.

2. Pellet according to claim 1 wherein the pharmaceutical salt of duloxetine is the hydrochloride salt.

3. Pellet according to any one of claims 1 to 2 wherein the intermediate non porous layer comprises copovidone or polyvinyl alcohol polymer as the water-soluble polymer.

4. Pellet according to any one of claims 1 to 3 wherein the water-soluble polymer of the intermediate layer is present in an amount ranging from 4 to 9% by weight.

5. Pellet according to any one of claims 1 to 4 wherein the enteric coating layer comprises methacrylic acid copolymer as the enteric polymer.

6. Pellet according to any one of claims 1 to 4 wherein the enteric coating layer further comprises pharmaceutically acceptable excipients.

7. Pellet according to claim 6 wherein one of the excipients is a plasticizer.

8. Pellet according to claim 7 wherein the plasticizer is triethyl citrate.

9. Pellet according to claim 8 wherein the triethyl citrate is present in amount ranging from 0.4 to 3% by weight.

10. Use of a pellet according to any one of claims 1 to 9 for manufacturing a pharmaceutical dosage form.

11. Pharmaceutical dosage form comprising a pellet according to any one of claims 1 to9.

12. Pharmaceutical dosage form comprising a pellet according to any one of claims 1 to 9 wherein the dosage form is a tablet or a capsule.

13. Process for manufacturing a pellet according to any one of claims 1 to 9 comprising:
a) preparing a core comprising duloxetine or a pharmaceutically acceptable salt thereof,
b) coating said core with an intermediate non porous layer comprising one or more film forming water-soluble polymers, and
c) coating said intermediate layer with an enteric coating layer comprising methacrylic acid copolymer as the enteric polymer.

14. Process according to claim 13 further comprising to apply a finishing layer after performing step c).

15. Process for manufacturing a pellet according any one of claims 13 or 14 wherein the preparation of the core in step a) comprises:
i) dissolving duloxetine or a pharmaceutically acceptable salt thereof in water, and
ii) spraying the solution obtained in i) on an inert bead,
wherein, steps i) and ii) are carried out at a temperature between 30°C and 50°C.
